# EUROPEAN PATENT SPECIFICATION

(11) **EP 1 713 327 B1**
(45) Date of publication and mention of the grant of the patent: **07.11.2007**
(21) Application number: 05708310.7
(22) Date of filing: 14.02.2005
(51) Int. Cl.: A01N 25/18, A01N 65/00, A01N 27/00, A61L 2/20, A62D 3/00

(54) **PROLONGED DEACTIVATION**
LANGZEITDESAKTIVIERUNG
DESACTIVATION PROLONGEE

(30) Priority: 13.02.2004 GB 0403219
(43) Date of publication of application: 25.10.2006
(73) Proprietor: Reckitt Benckiser (UK) Limited, Slough, Berkshire SL1 3UH (GB); UNIVERSITY OF SOUTHAMPTON, Highfield, Southampton SO17 1BJ (GB)
(72) Inventor: HIGGINS, Sabrina, Highfield Southampton SO17 1BJ (GB); HUGHES, John, Highfield Southampton SO17 1BJ (GB); MCKECHNIE, Malcolm, Tom, Dansom Lane, Hull HU8 7DS (GB)
(74) Representative: Bowers, Craig Malcolm
(86) International application number: PCT/GB2005/000485
(87) International publication number: WO 2005/079570

(56) References cited:
- EP-A- 0 682 942
- EP-A- 0 716 143
- EP-A- 0 843 965
- WO-A-01/76371
- WO-A-93/15774
- WO-A-99/15208
- WO-A-03/015522
- WO-A-20/05048718
- CA-A1- 2 222 911
- GB-A- 2 367 243
- US-A- 5 085 208
- US-A- 5 271 773
- "VAPORIZING DUST MITES" ENVIRONMENTAL HEALTH PERSPECTIVES, vol. 109, no. 8, August 2001 (2001-08), page A367, XP001202183 ISSN: 0091-6765
- "Cold Pressed Orange Oil" MATERIAL SAFETY DATA SHEET, [Online] January 2003 (2003-01), XP002332822 Retrieved from the Internet: URL:http://www.pdmchemicals.com/MSDS/MSDS- Orange%20Oil.pdf> [retrieved on 2005-06-20]
- S.BUDAVARI (ED.): "The Merck Index, Eleventh Edition" 1989, MERCK & CO., INC. , XP002332823 entry 7415: "beta-Pinene"
- DATABASE WPI Section Ch, Week 200367 Derwent Publications Ltd., London, GB; Class D16, AN 2003-701561 XP002332824 & JP 2003 180865 A (MITSUBISHI JUKOGYO KK) 2 July 2003 (2003-07-02)

## Description

The present invention relates to a method of deactivating dust mite allergens.

Various allergens are known to trigger a human reaction. For example, it has been known for a long time that house dust can trigger allergenic reactions in humans, such as asthma and rhinitis. It was reported, as early as 1928 that it was the dust mites in the dust that were the primary source of the allergenic response, but it was only in the 1960's that researchers appreciated its significance.

House dust mites produce detritus which causes allergenic reaction in many people. The major allergens are believed to be detritus from the mite species Dermatophogoides farinae and Dermatophagoides pteronyssinus (the allergens being known as Der f1 and Der p1 respectively). The detritus includes faeces as well as body part residues of the mites. A review is given in Experimental and Applied Acarology, 10 (1991) p. 167-186.

Other allergens which are problematic include cockroach allergens (notably the Bla g1 cockroach allergen), and cat allergens (Fel d1). In the case of cat allergens the coat/fur of the cat and/or its salivary deposits seem to be of significance in eliciting the allergenic response.

WO99/15208 describes a method for deactivating allergens derived from the D. Pteronyssinus and D. Farinae dust mite species, which comprises contacting the allergen with one of 28 deactivants which are described.

WO 01/76371 describes further deactivants for house dust mite allergens.

In accordance with a first aspect of the present invention there is provided a use of an allergen-reducing amount of an allergen-deactivating compound (hereinafter the "deactivant"), dispersed into an airspace at which an allergen-contaminated inanimate substrate is located, to achieve a prolonged reduction in the allergen loading of the substrate, wherein the reduction after 14 days, and preferably after 28 days is at least as great as the initial reduction.

We haves now determined that delivery of a deactivant into an airspace as described causes a permanent reduction in the population of allergens in an inanimate test source. By inanimate test source we mean a test source which is itself inanimate (eg it is not the skin or coat/fur of a live animal) and it does not contain living organisms, such as dust mites. Populations of dust mites would make any result difficult to interpret.

We have found that the reduction in allergen content in such a source is of long duration, at least 14 days, and suitably at least 28 days. Indeed, in tests we have carried out over a 28-day period, we have found that the allergen content may continue to decline over time, even though the deactivant may have been used days or weeks before. The results suggest that the allergenic species have been truly denatured or degraded, to the extent that, firstly, they cannot re-form, and secondly, their degradation products are not themselves allergenic. It further suggests that the action of the deactivant is not merely a masking or damping effect. Any such effect would be likely to break down over time.

Preferably the deactivant is selected from:
a terpene hydrocarbon;
a citrus oil;
a mint oil;
bois de rose oil;
oil of jasmine;
frankincense;
oil of bergamot; and
oil of lemon grass.

Preferred terpene hydrocarbons include tea tree oil, pinol and β-pinene.

An especially preferred deactivant is a citrus oil, most preferably orange oil.

Another especially preferred deactivant is β-pinene.

Use of the noun deactivant and the verb deactivate in this specification denote that some or all of a source of allergens at a locus are rendered unable to evoke an allergenic response in a human, by a method of the present invention. The net result is that the source may be reduced in its allergenicity, or its allergenicity may be completely removed.

A deactivant may suitably be a single compound. Alternatively a mixture of deactivants may be used together.

A deactivant may be part of a blend of compounds, not all of which are deactivants. For example a citrus oil is a blend of compounds not all of which will function as deactivants.

A deactivant may suitably be dispersed into the airspace over an extended period, for example at least 30 minutes, and preferably at least 1 hour.

A deactivant may suitably be dispersed into the airspace on two occasions, interrupted by a period in which there is no deactivant dispersal. A deactivant may be dispersed into the airspace on one or more further occasions, following a corresponding period or periods of no deactivant dispersal. Preferably each such dispersal occasion involves deactivant dispersal over an extended period, as described above. Preferably the or each period in which there is no deactivant dispersal is an extended period, for example at least 2 hours, preferably at least 4 hours, and most preferably at least 8 hours.

Preferably the dispersal of the deactivant into the airspace is as a vapour.

Preferably the dispersal of the deactivant into the airspace is aided by heat.

Preferably the heat applied to the deactivant may be by use of an oil burner, candle or hotplate. The use of a hotplate enables the heat applied to vaporise the deactivant to be controlled, in a manner which is not possible with prior methods. Preferably a hotplate is used, preferably having a temperature of at least 100°C.

Our work suggests that use of a hotplate below 100°C gives some allergen deactivating activity but that use of a higher temperature gives allergen deactivating activity of a substantially and surprisingly higher level, even though the quantity of deactivant dispersed may be the same in each case.

Preferably the hotplate has an electrical heat source.

Preferably a vessel containing the deactivant and the hotplate are in face-to-face contact. Preferably the hotplate has a flat surface and the vessel has a flat base, and the vessel rests on the hotplate. Preferably the vessel has an opening in its upper region. Preferably it has a fully open upper face. Preferably, therefore, the vessel has a flat base, a side (if cylindrical) or sides depending upwardly therefrom, and no further side.

Preferably the hotplate is at a temperature of at least 130°C.

Preferably the hotplate is at a temperature up to 300°C, preferably up to 250°C.

The deactivant may be used as such, or may be comprised within an oil-on-water formulation, or may be comprised within an oil-in-water emulsion formulation. Any oil/water formulation suitably comprises at least 0.5% by weight of the deactivant (in total, when more than one of said deactivants is employed), more preferably at least 2%, and most preferably at least 6%. Suitably any oil-water formulation comprises up to 25% by weight of the deactivant (in total, when more than one of said deactivants is employed), more preferably up to 20%, and most preferably up to 15%.

In this specification unless otherwise stated a percentage value given for a component denotes the weight of the component expressed as a percentage of the total weight of the formulation.

The formation of emulsions is generally well known in the art and is described, for example, in Modern Aspects of Emulsion Science, edited by Bernard P. Binks, The Royal Society of Chemistry, 1998 and Surfactant Science and Technology, Second Edition, Drew Myers, 1992, VCH Publishers, Inc. Non-ionic surfactants may be especially suitable. Proprietary surfactant packs may be employed to form emulsions, for example E-Z-MULSE (Trade Mark), a non-ionic surfactant pack from Florida Chemical Company, US.

The present invention involves the dispersal of an allergen deactivant into an airspace. It is possible that airborne allergens may be deactivated but it is believed that there is effective deactivation of allergens borne on surfaces within the airspace.

In accordance with a further aspect of the present invention there is provided the use of an allergen-deactivating compound in achieving irreversible reduction of an allergen population.

Preferably an allergen deactivated in a use in accordance with the present invention is a material which evokes an allergenic reaction in a human. For example it may be an allergen arising from house dust mites, or from pets. Most preferably use of this invention is able to deactivate, partially or wholly, an allergen arising from the mite species Dermatophogoides farinae (known as Der f1) or, especially from the mite species Dermatophagoides pteronyssinus (known as Der p1). Cat allergens (Fel d1) and cockroach allergens (Bla g1) may also be deactivated.

The present invention will be further described with reference to the following Examples.

### Experimental Protocol

When using house dust for allergen denaturing tests an inherent difficulty is the variability of the amount of allergen in each small sample, even when taken from the same dust reservoir. The amount of dust in the pre-treatment sample must be accurately estimated in order to determine the extent of any allergen denaturing. In these tests the dust sample was applied to the test exposure surface and then one half of this surface dust was removed to measure the control pre-treatment allergen level of that specific sample. Each control was directly relevant to each sample, which gave the best possible estimate of the level of allergen in the sample before exposure to possible denaturant. All tests employed a glass reinforced plastic booth of size 0.7m x 0.7m x 1.0m. All tests had 5 or 6 replicates. Average values are stated.

The following Examples all measure the reduction of the house dust mite Dermatophagoides pteronyssinus allergen - Der p1.

House dust was passed through a number of sieves and the fraction smaller than 53 µm was collected. 0.025g of dust was placed in a small sieve to distribute it evenly over the test surface. The test surface was a PTFE (polytetrafluoroethylene - trade mark TEFLON) coated metal tray of size 30cm x 30cm. The dust was applied to the tray by moving the sieve continuously over the surface while tapping the sieve. One half of the dust was then removed by suction onto an in-line filter and the weight recorded, this was the pre-treatment control. The tray was then placed in the booth. An oil burner containing 0.8ml of β-pinene floated on 6ml of distilled water was placed in the booth, and the booth was sealed. The oil burner candle was lit and allowed to burn, under the oil/water until all the liquid had been vaporised (approximately 30 minutes). The candle was then smothered. After 24 hours the tray was removed, the dust was collected from it and its weight recorded. The booth was washed with strong detergent between tests.

Two tests were carried out.

The test samples were assayed for the Der p1 allergen using an ELISA (enzyme linked immunosorbent assay) to determine the allergen content. This was then related to the weight of dust that had been present in each sample. All of the samples were multiplied up to compare the amount of allergen expected to be present in a 0.1g sample of dust. The percentage difference between the control sample and the exposed sample was then obtained. One analysis was carried out on week 0 (24 hours after the tests were completed. Another analysis was carried out after 2 weeks.

The Der p1 allergen reductions were as follows:
Week 0 - 68.0%
Week 2 - 66.3%

Statistical analysis suggested that these results could not be separated.

Corresponding tests were carried out and the same ELISA analysis carried out at Week 0 and after 4 weeks.

The Der p1 allergen reductions were as follows:
Week 0 - 77.5%
Week 4 - 91.6%

Statistical analysis suggested that the finding of higher allergen reduction after 4 weeks was statistically significant.

## Claims

1. The use of an allergen-reducing amount of an allergen-deactivating compound (hereinafter the "deactivant"), dispersed into an airspace at which an allergen-contaminated inanimate substrate is located, to achieve a prolonged reduction in the allergen loading of the substrate, wherein the reduction after 14 days is at least as great as the initial reduction.

2. The use as claimed in claim 1, wherein the reduction after 28 days is at least as great as the initial reduction.

3. The use as claimed in claim 1 or 2, wherein the deactivant dispersed into the airspace is as a vapour.

4. The use as claimed in claim 1 or 2, wherein the dispersal of the deactivant dispersed into the airspace is aided by heat.

5. The use as claimed in claim 4, wherein the heat applied to the deactivant is by use of an oil burner, candle or hotplate.

6. The use as claimed in any preceding claim, wherein the deactivant is dispersed into the airspace over a period of at least 30 minutes.

7. The use as claimed in any preceding claim, wherein the deactivant is selected from:
a terpene hydrocarbon;
a citrus oil;
a mint oil;
bois de rose oil;
oil of jasmine;
frankincense;
oil of bergamot; and
oil of lemon grass;
or a component thereof.

8. The use as claimed in any preceding claim, wherein the deactivant comprises a terpene hydrocarbon or a component thereof.

9. The use as claimed in any preceding claim, wherein the deactivant comprises β-pinene.

10. The use as claimed in any preceding claim, wherein the deactivant comprises orange oil or a component thereof.

## Patentansprüche

1. Verwendung einer Allergene reduzierenden Menge einer Allergene deaktivierenden Verbindung (hier nachstehend das "Deaktivierungsmittel"), das in einem Luftraum verteilt wird, in welchem sich ein mit Allergenen kontaminiertes lebloses Substrat befindet, um eine Langzeitreduzierung der Allergenbeladung des Substrats zu erzielen, wobei die Reduzierung nach 14 Tagen mindestens so viel wie die anfängliche Reduzierung beträgt.

2. Verwendung nach Anspruch 1, wobei die Reduzierung nach 28 Tagen mindestens so viel wie die anfängliche Reduzierung beträgt.

3. Verwendung nach Anspruch 1 oder 2, wobei das im Luftraum verteilte Deaktivierungsmittel ein Dampf ist.

4. Verwendung nach Anspruch 1 oder 2, wobei die Verbreitung des im Luftraum verteilten Deaktivierungsmittels durch Wärme unterstützt wird.

5. Verwendung nach Anspruch 4, wobei die auf das Deaktivierungsmittel aufgebrachte Wärme durch die Verwendung eines Ölbrenners, einer Kerze oder einer Heizplatte erfolgt.

6. Verwendung nach einem der vorangehenden Ansprüche, wobei das Deaktivierungsmittel über eine Dauer von mindestens 30 Minuten im Luftraum verteilt wird.

7. Verwendung nach einem der vorangehenden Ansprüche, wobei das Deaktivierungsmittel ausgewählt ist aus:
einem Terpenkohlenwasserstoff;
einem Zitrusöl;
einem Pfefferminzöl
Rosenholzöl;
Jasminöl;
Weihrauch;
Bergamotteöl; und
Zitronengrasöl;
oder einem Bestandteil davon.

8. Verwendung nach einem der vorangehenden Ansprüche, wobei das Deaktivierungsmittel einen Terpenkohlenwasserstoff oder einen Bestandteil davon umfasst.

9. Verwendung nach einem der vorangehenden Ansprüche, wobei das Deaktivierungsmittel β-Pinen umfasst.

10. Verwendung nach einem der vorangehenden Ansprüche, wobei das Deaktivierungsmittel Orangenöl oder einen Bestandteil davon umfasst.

## Revendications

1. Utilisation d'une quantité réductrice d'allergènes d'un composé désactivant les allergènes (ci-après le "désactivant"), dispersé dans un espace d'air où est situé un substrat inanimé contaminé par des allergènes, pour parvenir à une réduction prolongée de la charge d'allergènes du substrat, dans laquelle la réduction au bout de 14 jours est au moins aussi grande que la réduction initiale.

2. Utilisation suivant la revendication 1, dans laquelle la réduction au bout de 28 jours est au moins aussi grande que la réduction initiale..

3. Utilisation suivant la revendication 1 ou 2, dans laquelle le désactivant dispersé dans l'espace d'air est sous forme de vapeur.

4. Utilisation suivant la revendication 1 ou 2, dans laquelle la dispersion du désactivant dispersé dans l'espace d'air est facilitée par la chaleur.

5. Utilisation suivant la revendication 4, dans laquelle la chaleur appliquée au désactivant est fournie en utilisant à brûleur à huile, une bougie ou une plaque chauffante.

6. Utilisation suivant l'une quelconque des revendications précédentes, dans laquelle le désactivant est dispersé dans l'espace d'air en une période de temps d'au moins 30 minutes.

7. Utilisation suivant l'une quelconque des revendications précédentes, dans laquelle le désactivant est choisi entre :
un hydrocarbure terpénique ;
une essence d'agrume ;
une essence de menthe ;
l'essence de bois de rose ;
l'essence de jasmin ;
l'encens ;
l'essence de bergamote ; et
l'essence de citronnelle ;
ou un de leurs constituants.

8. Utilisation suivant l'une quelconque des revendications précédentes, dans laquelle le désactivant comprend un hydrocarbure terpénique ou un de ses constituants.

9. Utilisation suivant l'une quelconque des revendications précédentes, dans laquelle le désactivant le β-pinène.

10. Utilisation suivant l'une quelconque des revendications précédentes, dans laquelle le désactivant comprend l'essence d'orange ou un de ses constituants.
